# FASCICULE DE BREVET EUROPEEN

(11) **EP 3 621 596 B1**
(45) Date de publication et mention de la délivrance du brevet: **23.02.2022**
(21) Numéro de dépôt: 18738197.5
(22) Date de dépôt: 09.05.2018
(51) Int. Cl.: A61K 9/127, A61K 9/19, A61K 9/20, A61K 48/00, A61K 47/69, A61K 31/713, A61P 1/00, A61P 15/02

(54) **COMPRIMES DE VECTEURS D'ACIDES NUCLEIQUES**
NUKLEINSÄUREVEKTORTABLETTEN
TABLETS COMPRISING NUCLEIC ACID VECTORS

(30) Priorité: 10.05.2017 FR 1754105
(43) Date de publication de la demande: 18.03.2020
(73) Titulaire: Université de Bordeaux, 33000 Bordeaux (FR); Centre National de la Recherche Scientifique, 75016 Paris (FR); Institut Polytechnique de Bordeaux, 33400 Talence (FR); Ecole Nationale Supérieure d'Arts et Métiers, 75013 Paris (FR); Université de Paris, 75006 Paris (FR); Paris Sciences et Lettres, 75006 Paris (FR); Institut National de la Santé et de la Recherche Médicale, 75013 Paris (FR)
(72) Inventeur: BUSIGNIES-GODDIN, Virginie, 33700 Mérignac (FR); BIGEY, Pascal, 75009 Paris (FR); CHARRUEAU, Christine, 91200 Athis-Mons (FR); ESCRIOU, Virginie, 94800 Villejuif (FR); TCHORELOFF, Pierre, 33170 Gradignan (FR)
(74) Mandataire: Cabinet Becker et Associés
(86) Numéro de dépôt international: PCT/EP2018/062141
(87) Numéro de publication internationale: WO 2018/206723

(56) Documents cités:
- EP-A1- 1 920 765
- US-A1- 2011 028 535

## Description

### DOMAINE DE L'INVENTION

L'invention concerne un procédé de fabrication d'un comprimé comprenant un vecteur d'acide nucléique.

### ARRIERE PLAN TECHNOLOGIQUE DE L'INVENTION

La thérapie génique utilise des acides nucléiques (notamment ADN ou ARN) pour soigner ou prévenir des maladies. Selon la pathologie, l'acide nucléique est administré en vue de réguler, de réparer, de remplacer, d'ajouter ou de supprimer une séquence génétique. L'effet thérapeutique, prophylactique ou diagnostique dépend directement de la séquence d'acide nucléique ou du produit de l'expression génétique de cette séquence. Les acides nucléiques thérapeutiques comprennent par exemple les oligonucléotides antisens, les siRNA ou encore les shRNA. Les siRNA ou petits ARN interférents, notamment, couplés à des vecteurs constituent des substances actives à haut potentiel thérapeutique du fait de leur capacité à éteindre spécifiquement l'expression de protéines pathologiques. Les acides nucléiques thérapeutiques peuvent être injectés directement dans les cellules, sous forme d'acide nucléique nu, mais ils sont le plus souvent transportés dans les cellules du patient grâce à un vecteur d'acide nucléique. Les vecteurs d'acides nucléiques peuvent être des vecteurs viraux, ou bien des vecteurs synthétiques basés sur des systèmes polymériques ou lipidiques.

A l'heure actuelle, les vecteurs d'acides nucléiques se présentent sous forme de suspensions colloïdales administrées principalement par voie parentérale. Ce type de formulation ainsi que le mode d'administration imposent des contraintes fortes telles que la dimension des vecteurs administrés ou l'obtention de préparations stériles. Ces contraintes constituent un frein au développement des vecteurs d'acides nucléiques comme nouveaux candidats médicaments.

Il existe donc un besoin en nouvelles formulations de vecteurs d'acides nucléiques permettant d'éviter les contraintes citées tout en conservant l'activité biologique des acides nucléiques thérapeutiques.

### RESUME DE L'INVENTION

Selon un premier aspect, l'invention concerne un procédé de fabrication d'un comprimé comprenant un complexe entre :
(i) un acide nucléique; et
(ii) une particule lipidique comprenant un lipide cationique, la particule lipidique étant de préférence un liposome ou une micelle, de préférence un liposome ;
ledit procédé comprenant les étapes suivantes:
a) la dessiccation, notamment par lyophilisation, d'un mélange aqueux comprenant:
   - un complexe entre (i) un acide nucléique et (ii) une particule lipidique comprenant un lipide cationique, la particule lipidique étant de préférence un liposome ou une micelle, de préférence un liposome ;
   - un ou plusieurs excipient(s) adapté(s) à la dessiccation, notamment adaptés à la lyophilisation ;
b) le mélange d'un ou plusieurs excipient(s) de compression avec le mélange sec obtenu à l'étape a); et
c) la compression du mélange obtenu à l'étape b).

Dans un mode de réalisation particulier, ledit lipide cationique est choisi parmi les lipopolyamines, les ammoniums quaternaires, et les lipides ayant une tête cationique de type guanidine ou imidazole, de préférence le lipide cationique est choisi parmi le DiMyristylaminopropylaminopropyl (DMAPAP), le N-[1-(2,3-dioleyloxy)propyl]-N,N,N-trimethylammonium chloride (DOTMA), le [1,2-bis(oleoyloxy)-3-(trimethylammonio)propane] (DOTAP), le 3β[N-(N',N'-dimethylaminoethane)-carbamoyl] cholesterol (DCChol) et le dioctadecylamidoglycylspermine (DOGS), de préférence le Di-Myristylaminopropylaminopropyl (DMAPAP). Ledit lipide cationique peut éventuellement être en mélange avec un lipide neutre, notamment choisi parmi le 1,2-dioleoyl-sn-glycero-3-phosphoethanolamine (DOPE), le cholestérol, le dioleoyl-sn-glycero-3-phosphocholine (DOPC), le dipalmitoyl-sn-glycero-3-phosphocholine (DPPC) et le N'-(rac-1-[11-(F-octyl)undec-10-enyl]-2-(hexadecyl)glycero-3-phosphoethanoyl)-sperminecarboxamide), de préférence le lipide neutre est le 1,2-dioleoyl-sn-glycero-3-phosphoethanolamine (DOPE). En particulier, le mélange entre le lipide cationique et le lipide neutre peut comprendre entre 50% et 99% en mole de lipide cationique dans le mélange, de préférence environ 50% et entre 1% et 50% en mole de lipide neutre dans le mélange, de préférence environ 50%.

Selon un mode de réalisation particulier, le complexe comprend un acide nucléique thérapeutique, notamment un siRNA, un miRNA, un shRNA, un plasmide, ou un ARNm. Selon un mode de réalisation particulier, le rapport de charge (+/-) entre les charges positives du lipide cationique et les charges négatives de l'acide nucléique est compris entre 0,5 et 10, de préférence entre 4 et 10, de préférence environ 8.

Selon le procédé de l'invention, le ou les excipient(s) adapté(s) à la dessiccation, notamment la lyophilisation peuvent être des agents protecteurs (cryoprotecteurs ou lyoprotecteurs) et/ou des agents de charge, notamment choisis parmi le tréhalose, le mannitol, le saccharose, le sorbitol, le lactose, le glucose, le glycérol, la glycine, l'alanine, la lysine, le polyéthylène glycol, la poly vinyl pyrrolidone et le dextran, éventuellement en mélange. En particulier, l'excipient adapté à la lyophilisation est le tréhalose, notamment en une proportion comprise entre 10% et 100% en masse par rapport à la masse d'excipient(s) adapté(s) à la lyophilisation, éventuellement mélangé avec un autre excipient adapté à la lyophilisation, de préférence le mannitol, notamment en une proportion comprise entre 0 et 90% en masse par rapport à la masse d'excipient(s) adapté(s) à la lyophilisation.

Selon le procédé de l'invention, le ou les excipient(s) de compression permettent de protéger le complexe entre l'acide nucléique et la particule lipidique comprenant le lipide cationique durant l'étape c) de compression, lesdits excipients de compression étant en particulier un lubrifiant (par exemple le stéarate de magnésium, l'acide stéarique et le talc, de préférence le stéarate de magnésium), éventuellement mélangé avec un autre excipient de compression, de préférence un diluant (par exemple le lactose, l'amidon, le phosphate de calcium et ses dérivés et la cellulose et ses dérivés, de préférence le lactose). En particulier, le lubrifiant peut représenter de 0,25 à 5% en masse du comprimé fini, de préférence 0,5%.

Dans un mode particulier de réalisation, le procédé selon l'invention comprend une étape de compression (étape c) réalisée à une pression comprise entre 10 et 400 MPa, de préférence entre 50 et 250 MPa.

Selon un autre aspect, l'invention concerne un comprimé susceptible d'être obtenu au moyen du procédé précédemment décrit. Le comprimé de l'invention peut comprendre :
- un complexe entre un acide nucléique et un liposome ou une micelle comprenant un lipide cationique;
- un ou plusieurs excipients de lyophilisation appropriés permettant de protéger ledit complexe durant l'étape a) de lyophilisation et/ou permettant d'obtenir un lyophilisat de texture adaptée pour l'étape de compression, de préférence du tréhalose et du mannitol.
- un ou plusieurs excipients de compression appropriés permettant de protéger ledit complexe durant l'étape c) de compression et de conserver l'activité biologique du complexe.

Selon un mode de réalisation particulier, le comprimé de l'invention est destiné à une administration par voie orale, vaginale, rectale, sublinguale ou transmucosale, de préférence par voie orale.

La présente invention concerne également le comprimé précédemment décrit pour une utilisation dans le traitement des pathologies du tractus gastro-intestinal ou des pathologies vaginales.

### LEGENDES DES FIGURES

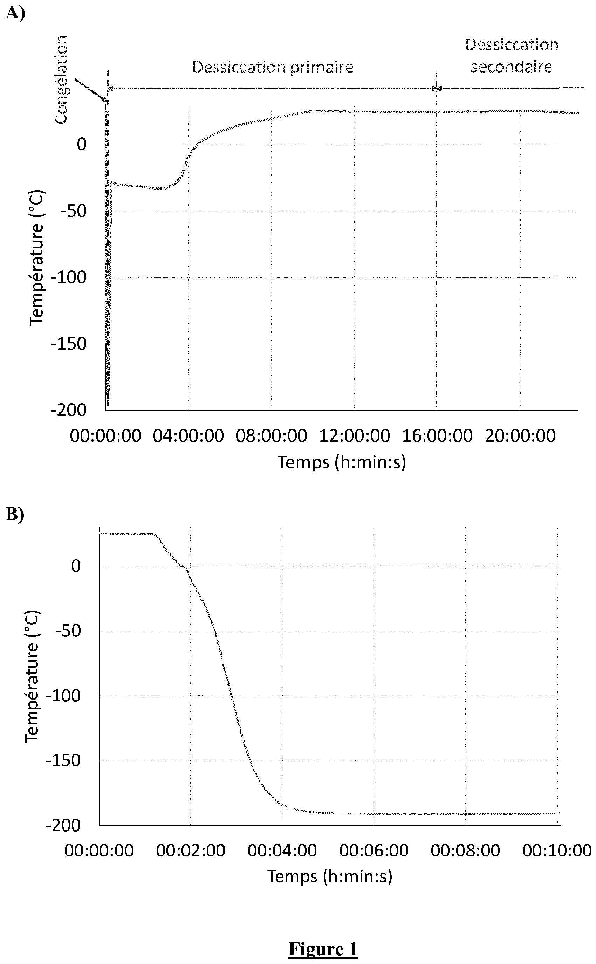

**Figure 1****. Cycle de lyophilisation :** Représentation de la température du produit au cours des trois étapes du cycle ; (a) congélation, dessiccation primaire et début de la dessiccation secondaire ; (b) zoom sur la congélation dans l'azote liquide.

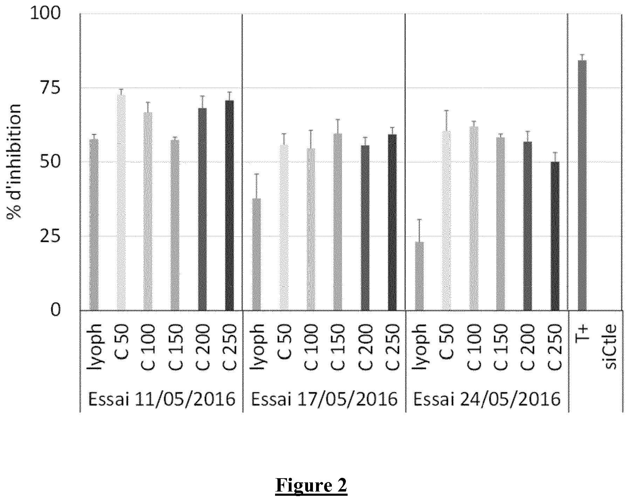

**Figure 2****. Efficacité des lyophilisats (=lyoph) et des comprimés contenant des lipoplexes en fonction de la contrainte de compression appliquée au cours du procédé de compression (CX avec X = 50, 100, 150, 200 ou 250 MPa) :** Transfection sur cellules B16Luc pendant 48h, en triplicat, 0,5µg de siRNA luciférase par puits. Dosage de la luciférase et expression du pourcentage d'inhibition par rapport à des cellules non transfectées. Témoin positif (T+) : cellules transfectées avec des lipoplexes préparés extemporanément (non lyophilisés et non comprimés), témoin négatif (siCtle) : cellules transfectées avec des lipoplexes contenant un siRNA contrôle, remis en suspension depuis un comprimé.

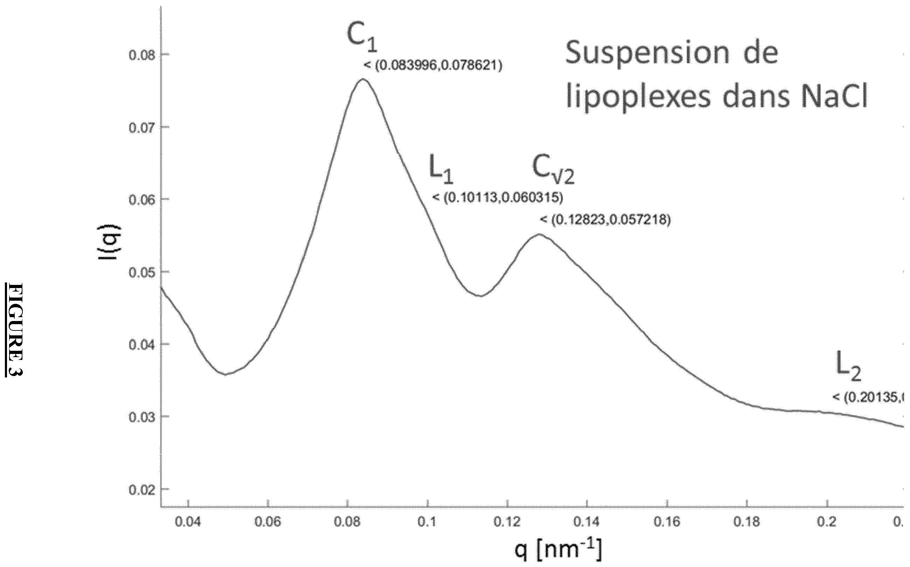

**Figure 3** **:** Profil SAXS de lipoplexes de siRNA (DMAPAP/DOPE + Alginate), préparés à un rapport de charge +/- de 8 dans un tampon NaCl 150mM. L'analyse des pics permet d'identifier deux phases, une majoritaire cubique (C₁, q=0,084 nm⁻¹) et une lamellaire minoritaire (Li, q=0,101 nm⁻¹).

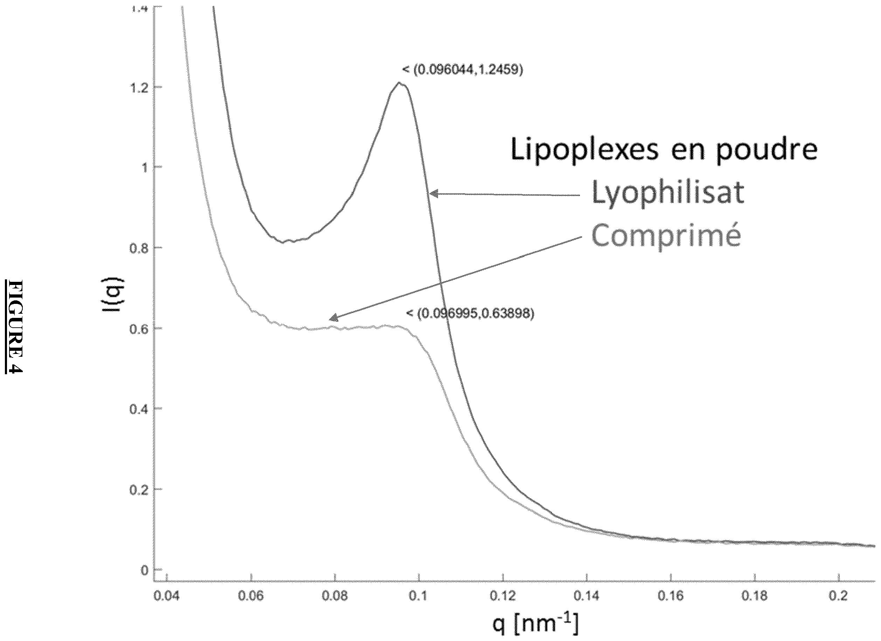

**Figure 4** : Profil SAXS de lipoplexes de siRNA sous forme sèche, en poudre, ayant subi une étape de lyophilisation puis une étape de compression. Les lipoplexes de siRNA (DMAPAP/DOPE + Alginate), préparés à un rapport de charge +/- de 8 dans un tampon NaCl 150mM sont supplémentés en tréhalose et maltose puis lyophilisés pendant 30h. Le lyophilisat est pesé, supplémenté en lactose et comprimé. L'analyse SAXS est réalisée sur de la poudre de lyophilisât ou de comprimé placée dans un capillaire. On détecte un pic (q=0,096 nm⁻¹) unique et large.

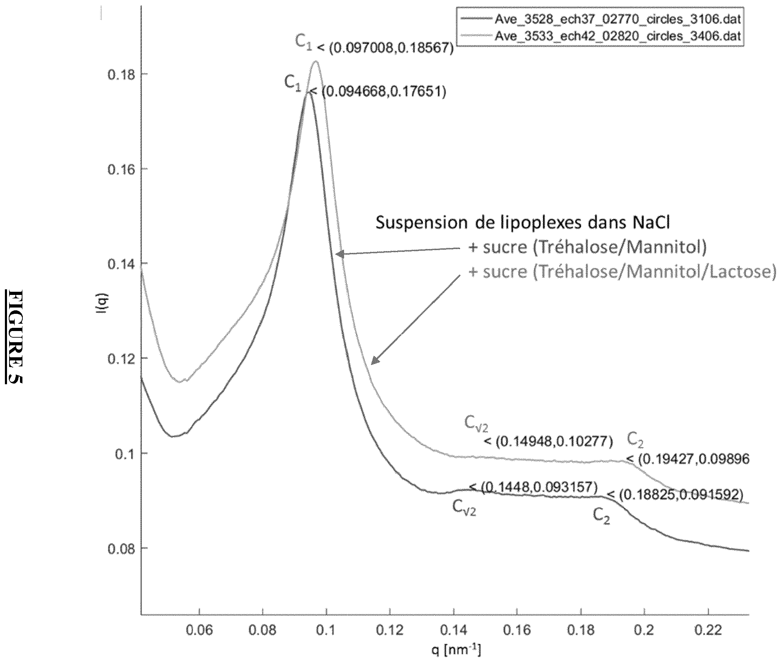

**Figure 5** : Profil SAXS de lipoplexes de siRNA en suspension dans NaCl/ sucre. Les lipoplexes préparés comme indiqué dans la légende de la Figure 3 sont supplémentés par un premier mélange Tréhalose / Mannitol ou deuxième mélange Tréhalose / Mannitol / Lactose dans les mêmes conditions que les échantillons lyophilisés ou comprimés respectivement. L'analyse des pics permet d'identifier une seule phase cubique dont la position du premier pic est légèrement décalée entre le tracé du premier mélange (C₁, q=0,095 nm⁻¹) et le tracé du deuxième mélange (C₁, q=0,097 nm⁻¹).

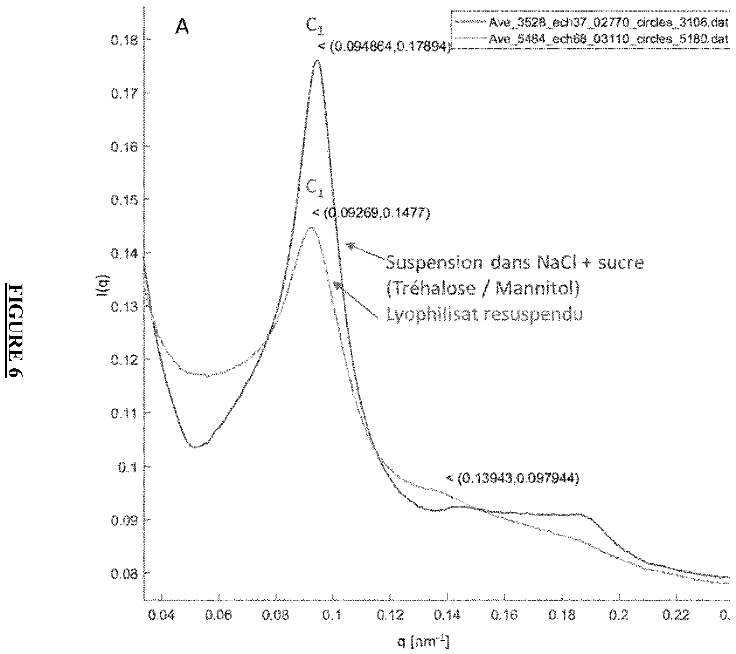

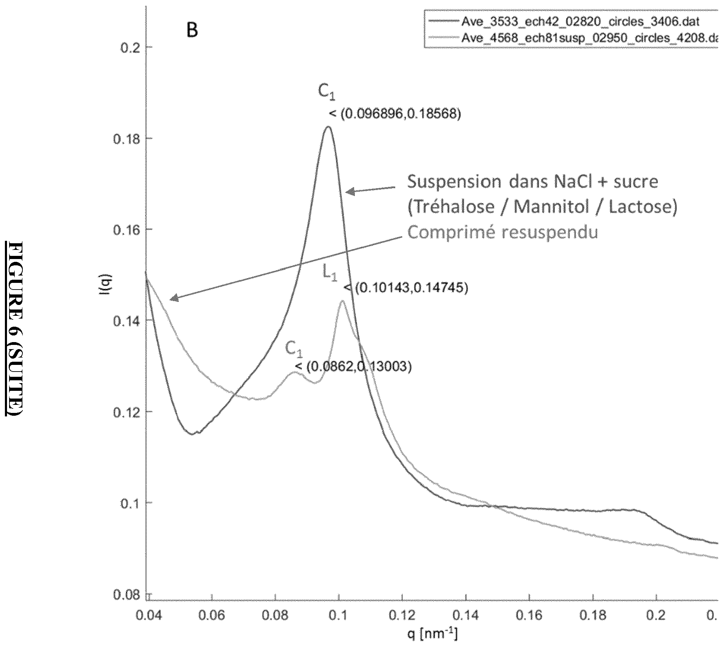

**Figure 6** : Profil SAXS de lipoplexes de siRNA en suspension. La première courbe correspond aux échantillons ayant subi (A) une lyophilisation puis (B) une compression (voir légende Figure 4) puis une remise en suspension par ajout d'eau. Une deuxième courbe correspond à l'échantillon en suspension n'ayant pas subi de lyophilisation ni de compression mais supplémenté avec la même quantité de (A) tréhalose / mannitol que l'échantillon lyophilisé resuspendu ou (B) tréhalose / mannitol / lactose que l'échantillon compressé resuspendu. L'analyse des pics permet d'identifier une seule phase dans le tracé correspondant à la deuxième courbe, de type cubique (C₁, (A) q=0,094 nm⁻¹ ; (B) q=0,096 nm⁻¹), une phase cubique dans le tracé de la première courbe en (A) (C₁, q=0,093 nm⁻¹) et deux phases dans le tracé de la première courbe en (B), une cubique (C₁, q=0,086 nm⁻¹) et une lamellaire (L₁, q=0,101 nm⁻¹).

### DESCRIPTION DETAILLEE DE L'INVENTION

Les inventeurs proposent la mise en forme de vecteurs d'acides nucléiques sous forme de comprimés en vue de leur administration par voie orale, vaginale, rectale, sublinguale ou transmucosale. La forme « comprimé » permet d'avoir accès à une administration facile, tout en s'affranchissant des contraintes de formulation et d'administration évoquées ci-dessus. Par ailleurs, le comprimé donne accès à de nombreuses possibilités de formulation permettant de contrôler ou de moduler la libération des vecteurs d'acides nucléiques en fonction des cibles thérapeutiques visées. La forme « comprimé » représente également une avancée pour la stabilité des vecteurs d'acides nucléiques. Elle permet notamment de s'affranchir de leur préparation extemporanée. De plus, la forme « comprimé » améliore le confort des patients et l'observance des traitements.

Or, avant la mise en œuvre de la présente invention, aucun comprimé de vecteur d'acides nucléiques n'était disponible ni même envisageable, l'homme du métier craignant une perte d'activité des acides nucléiques au cours des étapes nécessaires à la formation d'un comprimé. Les inventeurs proposent toutefois, à l'encontre de ce préjugé, un moyen innovant de production de comprimés de vecteurs d'acides nucléiques.

L'invention concerne un procédé de fabrication d'un comprimé comprenant au moins un vecteur d'acide nucléique. Plus particulièrement, l'invention proposée permet de mettre sous une forme « comprimé » des vecteurs d'acides nucléiques, tout en conservant leurs propriétés thérapeutiques malgré les stress mécaniques associés à cette opération de mise en forme.

### Définitions

Par « comprimé », on entend toute formulation solide homogène sans forme ou dimension déterminées, obtenue à partir de poudre comprimée, la poudre pouvant ou non être agglomérée.

Par « vecteur d'acide nucléique » (ou encore le « complexe », ci-dessous), on entend toute structure connue comme adaptée pour vectoriser un acide nucléique ; en particulier, on entend par ces termes un complexe entre (i) un acide nucléique et (ii) une particule lipidique comprenant un lipide cationique, la particule lipidique étant de préférence un liposome ou une micelle, de préférence un liposome.

Par « lipoplexe » on entend un complexe entre (i) un acide nucléique et (ii) un liposome comprenant un lipide cationique.

Par « lipide cationique » on entend un lipide possédant une charge globale positive. Un lipide cationique comprend une tête polaire cationique et une ou plusieurs chaîne(s) hydrophobe(s).

Par « lipide neutre » on entend un lipide dont la charge globale est neutre, plus particulièrement un lipide zwitterionique.

Par « micelle » on entend un agrégat sphéroïdal de molécules amphiphiles possédant une tête polaire hydrophile et une chaîne hydrophobe.

On entend par « liposome » une vésicule artificielle formée par des bicouches lipidiques concentriques, emprisonnant entre elles des compartiments aqueux.

Par exemple, le vecteur d'acide nucléique peut être choisi parmi les liposomes, les lipides, par exemple des lipides cationiques, des lipides anioniques, des lipides amphotères ou des lipides non chargés, des polymères cationiques, des polymères, des hydrogels, des micro- or nano-capsules (biodégradables), des microsphères (éventuellement bioadhésives), des cyclodextrines, des vecteurs protéiques, ou n'importe quelle combinaison de tels éléments. De préférence, le vecteur peut être choisi parmi les systèmes de délivrance basés sur des lipides, les systèmes de délivrance basés sur des polyimines, des dendrimères, des particules de PLGA (poly(lactide-co-glycolide)), les systèmes décrits dans WO15023775, et systèmes similaires.

Par « environ » est entendu la valeur + ou - 10 %, de préférence + ou - 5 % de celle-ci. Par exemple, environ 50 signifie de 45 à 55, et de préférence de 47,5 à 52,5.

### Préparation du vecteur d'acide nucléique

L'acide nucléique peut notamment être de l'acide désoxyribonucléique (ADN) ou de l'acide ribonucléique (ARN), de toute taille, simple brin ou double brin, linéaire ou circulaire, non modifié ou modifié chimiquement (telles que les formes phosphorothioate, phosphoroamidate et/ou 2'Omethyl). La taille de l'acide nucléique peut varier dans une large mesure, par exemple de 10 paires de bases à plusieurs dizaines de milliers de paires de bases. Par exemple, la taille de l'acide nucléique peut atteindre environ 10000 paires de bases, voire plus, dans le cas d'un plasmide, environ 19-25 nucléotides dans le cas d'un siRNA double brin ou environ 20-30 nucléotides dans le cas d'un microARN simple brin. Il peut s'agir de séquences naturelles, hybrides ou synthétiques, d'une origine quelconque (procaryote, eucaryote, virus, parasite, plante...). De préférence, l'acide nucléique est un acide nucléique thérapeutique, c'est-à-dire un acide nucléique qui possède un effet thérapeutique via la régulation, la réparation, le remplacement, l'ajout ou la suppression d'une séquence génétique. De préférence, l'acide nucléique peut moduler l'expression d'une protéine et peut être notamment choisi parmi des oligonucléotides antisens, des oligonucléotides pour le saut d'exon, des oligonucléotides pour la modification d'épissage alternatif, des ARNs interférents, des ARN messagers ou de l'ADN plasmidique. De préférence, l'acide nucléique est un ARN interférent. Dans le contexte de l'invention, un « ARN interfèrent » est un ARN qui permet d'inhiber l'expression génique. Plus particulièrement, un ARN interférent est un acide ribonucléique de petite taille (notamment entre 20 et 25 paires de bases, en particulier entre 21 et 23 paires de bases) qui interfère avec un ARN messager spécifique, conduisant à sa dégradation et à l'inhibition de sa traduction en protéine. Par exemple, les siRNA (small interfering RNA) ou miRNA (micro RNA) sont des ARN interférents.

En particulier, l'acide nucléique est sélectionné parmi un plasmide, un siRNA, un miRNA, un ARNm et un shRNA, de préférence l'acide nucléique est un siRNA ou un miRNA. De préférence, l'acide nucléique est un siRNA.

Le comprimé fabriqué selon l'invention comprend un ou plusieurs vecteur(s) d'acide nucléique (ou complexe(s)), tel(s) que défini(s) ci-dessus. Selon un mode particulier de réalisation, le comprimé comprend une combinaison de plusieurs vecteurs d'acide nucléique différents, notamment une combinaison d'au moins 2, au moins 3, au moins 4, voire au moins 5 vecteurs d'acide nucléique différents. Selon un autre mode particulier de réalisation, le comprimé comprend une combinaison d'un ou plusieurs vecteur(s) d'acide nucléique (ou complexe(s)) avec au moins un probiotique.

On entend par « probiotique » un micro-organisme vivant qui, lorsqu'il est administré en quantité suffisante, exerce des effets positifs sur la santé, au-delà des effets nutritionnels traditionnels. Les effets positifs sur la santé incluent par exemple une amélioration de la digestion ou des défenses immunitaires.

Selon la présente invention, un complexe est formé par interactions électrostatiques entre un acide nucléique chargé négativement et une particule lipidique comprenant un lipide cationique.

De manière préférée, la particule lipidique comprenant un lipide cationique est une micelle ou un liposome.

De manière encore plus préférée, la particule lipidique est un liposome. Selon ce mode de réalisation, le complexe formé entre l'acide nucléique et le liposome est appelé « lipoplexe ».

Selon un mode de réalisation particulier, l'invention concerne un procédé de fabrication d'un comprimé comprenant un complexe entre :
(i) un acide nucléique; et
(ii) une particule lipidique comprenant un lipide cationique, ladite particule lipidique étant de préférence un liposome ou une micelle, de manière encore plus préférée un liposome.

Selon un mode particulier de réalisation, le procédé de l'invention peut comprendre, avant l'étape de dessiccation décrite en détails ci-dessous, une étape de formation d'un complexe entre l'acide nucléique et la particule lipidique comprenant un lipide cationique, la particule lipidique étant de préférence un liposome ou une micelle, de préférence un liposome.

Selon un mode de réalisation particulier, l'acide nucléique peut être pré-mélangé à un polymère anionique, préalablement à l'étape de formation du complexe entre l'acide nucléique et la particule lipidique. Ledit polymère anionique est un adjuvant permettant d'améliorer la structuration du complexe entre l'acide nucléique et la particule lipidique, et peut ainsi augmenter l'efficacité dudit complexe. Le polymère anionique peut être choisi parmi les polysaccharides anioniques et les polypeptides anioniques, de préférence le polymère anionique est choisi parmi l'alginate de sodium et le polyglutamate de sodium. Selon un mode particulier de réalisation, le polymère anionique est un alginate, notamment l'alginate de sodium. Ainsi, le procédé de fabrication selon l'invention peut comprendre en outre une étape de mélange entre l'acide nucléique et un polymère anionique, préalablement à l'étape de formation du complexe entre l'acide nucléique et la particule lipidique.

En outre, le procédé selon l'invention peut comprendre une étape préalable de formation de la particule lipidique comprenant un lipide cationique, la particule lipidique étant de préférence un liposome ou une micelle, de préférence un liposome.

De préférence, le lipide cationique est choisi parmi les lipides cationiques capables de former une particule par association des chaînes hydrophobes de plusieurs lipides. Le lipide cationique selon l'invention peut être choisi parmi les lipopolyamines, les ammoniums quaternaires, et les lipides ayant une tête cationique de type guanidine ou imidazole. De préférence le lipide cationique est choisi parmi le Di-Myristylaminopropylaminopropyl (ou DMAPAP), le N-[1-(2,3-dioleyloxy)propyl]-N,N,N-trimethylammonium chloride (ou DOTMA), le [1,2-bis(oleoyloxy)-3-(trimethylammonio)propane] (ou DOTAP), le 3β[N-(N',N-dimethylaminoethane)-carbamoyl] cholesterol (DCChol), et le dioctadecylamidoglycylspermine (ou DOGS). De préférence, le lipide cationique est le Di-Myristylaminopropylaminopropyl (ou DMAPAP).

Selon un mode de réalisation particulier, le lipide cationique est mélangé avec un lipide neutre. De préférence, le lipide neutre est choisi parmi le 1,2-dioleoyl-sn-glycero-3-phosphoethanolamine (DOPE), le Cholestérol, le dioleoyl-sn-glycero-3-phosphocholine (DOPC), le dipalmitoyl-sn-glycero-3-phosphocholine (DPPC) et le N'-(rac-1-[11-(F-octyl)undec-10-enyl]-2-(hexadecyl)glycero-3-phosphoethanoyl)-sperminecarboxamide). De préférence le lipide neutre est le 1,2-dioleoyl-sn-glycero-3-phosphoethanolamine (DOPE).

Ainsi, le comprimé selon l'invention peut comprendre un complexe entre (i) un acide nucléique et (ii) une particule lipidique comprenant un lipide cationique éventuellement mélangé avec un lipide neutre.

Selon un mode de réalisation préféré, le comprimé selon l'invention comprend un complexe entre (i) un acide nucléique et (ii) une particule lipidique comprenant un mélange entre un lipide cationique et un lipide neutre. De préférence, la particule lipidique est une micelle ou un liposome, de manière préférée un liposome.

Le mélange entre le lipide cationique et le lipide neutre comprend notamment une proportion définie de lipide cationique et de lipide neutre. De préférence, la proportion de lipide cationique est comprise entre 50% et 99% en mole de lipide dans le mélange. Notamment, la proportion de lipide cationique peut être d'environ 50%, environ 60%, environ 70%, environ 80% ou environ 90% en mole de lipide dans le mélange. De manière préférée, la proportion de lipide cationique est d'environ 50% en mole de lipide dans le mélange.

De préférence, la proportion de lipide neutre est comprise entre 1% et 50% en mole de lipide dans le mélange. Notamment, la proportion de lipide neutre peut être d'environ 10%, environ 20%, environ 30%, environ 40% ou environ 50% en mole de lipide dans le mélange. De manière préférée, la proportion de lipide neutre est d'environ 50% en mole de lipide dans le mélange.

Selon un mode de réalisation préféré, le lipide cationique est mélangé avec le lipide neutre selon un ratio équimolaire 1:1.

Le procédé selon l'invention peut comprendre une étape de mélange entre le lipide cationique et le lipide neutre, préalablement à l'étape de formation de la particule lipidique, la particule lipidique étant ensuite utilisée durant l'étape de formation du complexe avec l'acide nucléique.

Selon un mode particulier de réalisation, le mélange entre un lipide cationique et un lipide neutre est un mélange entre le DMAPAP et le DOPE.

Selon un mode de réalisation particulier, l'acide nucléique de l'invention est mélangé avec la particule lipidique de l'invention selon un rapport de charge défini. Le « rapport de charge » correspond au rapport +/- entre le nombre de charges positives du lipide cationique et le nombre de charges négatives de l'acide nucléique. Ce rapport de charge peut influencer l'efficacité du complexe formé en jouant sur les propriétés physico-chimiques (par exemple la taille ou la charge de surface). Ledit rapport de charge peut varier et être adapté par l'homme du métier, en fonction du lipide et de l'acide nucléique sélectionnés ainsi que de l'application souhaitée (organe ou type cellulaire ciblé). De préférence le rapport de charge +/- est compris entre 0,5 et 10, par exemple environ 1, 2, 3, 4, 5, 6, 7, 8, 9 ou 10. De préférence le rapport de charge +/- est compris entre 4 et 10, par exemple environ 4, 5, 6, 7, 8, 9 ou 10. De manière encore plus préférée, le rapport de charge est d'environ 8.

Selon un mode de réalisation particulier, le mélange entre l'acide nucléique et la particule lipidique est un mélange aqueux. Ainsi, le procédé selon l'invention peut comprendre une étape de préparation d'un mélange aqueux entre l'acide nucléique et la particule lipidique comprenant un lipide cationique.

De préférence, le mélange aqueux entre l'acide nucléique et la particule lipidique est réalisé dans une solution tampon. En particulier, le pH de ladite solution tampon est d'environ 4 à 8. De manière préférée la solution tampon choisie est une solution comprenant du NaCl. Ladite solution tampon peut notamment comprendre une concentration en NaCl d'environ 150mM. Par exemple, l'homme du métier peut utiliser une solution tampon de type HEPES ou opti-MEM, ou une solution comprenant du glucose.

Ainsi, selon un mode particulier de réalisation, le procédé de fabrication d'un comprimé selon l'invention peut comprendre :
- une étape de formation de la particule lipidique, de préférence du liposome, dans une solution tampon ; et/ou
- une étape de préparation de l'acide nucléique dans une solution tampon ; et/ou
- une étape de mélange de l'acide nucléique et de la particule lipidique dans une solution tampon.

Selon un mode de réalisation particulier, le temps de mélange entre l'acide nucléique et la particule lipidique, de préférence un liposome, est compris entre 10 secondes et plusieurs jours, par exemple entre 10 secondes et 10 heures, notamment entre 5 minutes et 5 heures, en particulier entre 10 min et 1 heure. De préférence, le temps de mélange est supérieur ou égal à 30 minutes, notamment égal à environ 30 minutes. Le mélange peut être réalisé dans une large gamme de température. Toutefois, selon un mode préféré de réalisation, le mélange est réalisé à une température comprise entre 10°C et 30°C, notamment entre 15°C et 25°C.

Après formation du complexe, ce dernier peut être soumis directement aux étapes suivantes du procédé selon l'invention, ou conservé, notamment pendant au moins 1 jour, avant lesdites étapes suivantes.

### Formulation en « comprimé »

Le procédé selon l'invention comprend une phase de formulation en comprimé à partir du mélange aqueux comprenant l'acide nucléique et la particule lipidique tel que décrit ci-dessus. Les inventeurs de la présente demande sont parvenus à mettre sous forme « comprimé » des vecteurs d'acides nucléiques (ou complexes), en vue d'une éventuelle administration par voie orale, vaginale, rectale, sublinguale ou transmucosale, tout en conservant les propriétés biologiques desdits vecteurs d'acides nucléiques.

Plus particulièrement, le procédé selon l'invention comprend les étapes de :
- dessiccation du mélange aqueux comprenant un complexe tel que défini ci-dessus; et
- compression du mélange sec ainsi obtenu.

De préférence, le procédé selon l'invention comprend les étapes suivantes :
a) la dessiccation d'un mélange aqueux comprenant:
   - le complexe tel que défini ci-dessus ; et
   - un ou plusieurs excipient(s) adapté(s) à la dessiccation
b) le mélange d'un ou plusieurs excipient(s) de compression avec le mélange sec obtenu à l'étape a); et
c) la compression du mélange obtenu à l'étape b).

### • Etape a) de dessiccation :

On entend par dessiccation, tout procédé de déshydratation, capable d'éliminer l'eau contenue dans une substance ou un corps. Par exemple, la dessiccation peut être réalisée par séchage par atomisation, par séchage convectif ou par lyophilisation. Selon un mode de réalisation préféré, la dessiccation est réalisée par lyophilisation. Selon ce mode de réalisation, le ou les excipient(s) adapté(s) à la dessiccation sont des excipients adaptés à la lyophilisation. La lyophilisation est un procédé de dessiccation sous vide, à basse température, permettant l'élimination par sublimation de l'eau contenue dans un produit préalablement congelé.

Selon un mode de réalisation préféré, l'étape a) de dessiccation est une lyophilisation. Selon un mode particulier de réalisation, la lyophilisation mise en œuvre comprend les étapes suivantes :
- une congélation du mélange aqueux comprenant un complexe tel que défini ci-dessus et un ou plusieurs excipient(s) adapté(s) à la lyophilisation;
- une dessiccation primaire (ou sublimation); et
- une dessiccation secondaire.

De préférence, le procédé selon l'invention comprend, préalablement à l'étape de dessiccation, une étape de mélange du complexe tel que défini ci-dessus avec le ou les excipient(s) adapté(s) à la dessiccation.

Le ou les excipient(s) adapté(s) à la dessiccation sont des excipients capables de préserver la structure des vecteurs d'acides nucléiques durant l'étape de dessiccation, permettant ainsi de préserver l'activité biologique de l'acide nucléique. Le ou les excipient(s) adapté(s) à la dessiccation permettent également d'obtenir un mélange sec adapté à l'étape de compression. Plus particulièrement, le ou les excipient(s) permettent d'obtenir un produit sec dont la texture (influencée par l'humidité résiduelle) est adaptée à l'étape de compression. Le ou les excipient(s) permettent également d'obtenir un produit sec dont la masse et/ou la résistance mécanique sont adaptées à l'étape de compression, lesdits excipients permettant par exemple d'éviter un effondrement de la structure du produit sec. De préférence, ledit ou lesdits excipient(s) adapté(s) à la dessiccation, de préférence à la lyophilisation, facilite(nt) le broyage puis le mélange homogène du produit sec obtenu avec le ou les excipient(s) de compression. L'optimisation des paramètres tels que la texture, la masse et/ou la résistance mécanique du produit sec par le choix de la nature et de la quantité des excipients adaptés à la dessiccation est classiquement réalisée par l'homme du métier, notamment en fonction des caractéristiques physicochimiques de chacun des excipients (existence d'eutectiques, de températures de transition vitreuse, ...).

Selon le mode préféré de réalisation de l'invention, mettant en œuvre une lyophilisation à l'étape a), le ou les excipient(s) adapté(s) à la dessiccation sont adaptés à la lyophilisation. Ainsi, le ou les excipient(s) adapté(s) à la lyophilisation permettent de protéger le complexe durant l'étape de lyophilisation et/ou permettent d'obtenir un lyophilisat de texture adaptée pour l'étape de compression.

Selon un mode de réalisation particulier, le ou les excipient(s) adapté(s) à la lyophilisation sont des agents protecteurs (cryoprotecteurs ou lyoprotecteurs) et/ou des agents de charge (ou agents de ballast). Des excipients cryoprotecteurs sont des excipients qui protègent les structures des vecteurs d'acides nucléiques durant l'étape de congélation. Des excipients lyoprotecteurs sont des excipients qui protègent les structures des vecteurs d'acides nucléiques durant l'étape de déshydratation. Au cours de la lyophilisation, ces agents se substituent aux molécules d'eau pour interagir avec les têtes polaires des phospholipides (hypothèse de remplacement de l'eau) et forment une matrice vitreuse qui contribue à maintenir la structure des lipoplexes (hypothèse de la vitrification). Des agents de charge (ou agents de ballast) sont des excipients qui permettent de maintenir la structure et la rigidité du lyophilisat (par opposition à un état effondré).

Selon un mode de réalisation particulier, le ou les excipient(s) adapté(s) à la lyophilisation sont choisis parmi le tréhalose, le mannitol, le saccharose, le sorbitol, le lactose, le glucose, le glycérol, la glycine, l'alanine, la lysine, le polyéthylène glycol, la poly vinyl pyrrolidone (PVP) et le dextran, éventuellement en mélange.

Selon un mode particulier de réalisation, l'excipient adapté à la lyophilisation est le tréhalose, en une proportion comprise entre 1% et 100%, notamment entre 10% et 100%, en masse par rapport à la masse d'excipient(s) adapté(s) à la lyophilisation. Par exemple, la proportion de tréhalose est d'environ 10%, environ 20%, environ 30%, environ 40%, environ 50%, environ 60%, environ 70%, environ 80%, environ 90% ou environ 100% en masse par rapport à la masse d'excipient(s) adapté(s) à la lyophilisation.

Selon un mode particulier de réalisation, l'excipient adapté à la lyophilisation est le mannitol, en une proportion comprise entre 1% et 90% en masse par rapport à la masse d'excipient(s) adapté(s) à la lyophilisation. Par exemple, la proportion de mannitol est d'environ 0%, environ 10%, environ 20%, environ 30%, environ 40%, environ 50%, environ 60%, environ 70%, environ 80% ou environ 90% en masse par rapport à la masse d'excipient(s) adapté(s) à la lyophilisation.

De préférence, l'excipient adapté à la lyophilisation est le tréhalose, éventuellement mélangé avec un autre excipient adapté à la lyophilisation choisi parmi le mannitol, le saccharose, le sorbitol, le lactose, le glucose, le glycérol, la glycine, l'alanine, la lysine, le polyéthylène glycol, la poly vinyl pyrrolidone (PVP) et le dextran, de préférence le mannitol.

Selon un mode de réalisation particulier, le tréhalose est mélangé avec le mannitol selon des proportions définies. En particulier, la proportion de tréhalose peut être comprise entre 10% et 99% en masse par rapport au mélange tréhalose/mannitol. Par exemple, la proportion de tréhalose est d'environ 10%, environ 20%, environ 30%, environ 40%, environ 50%, environ 60%, environ 70%, environ 80% ou environ 90% en masse par rapport au mélange tréhalose/mannitol, de préférence environ 71% en masse par rapport au mélange tréhalose/mannitol.

Selon un mode de réalisation particulier, la proportion de mannitol peut être comprise entre 1% et 90% en masse par rapport au mélange tréhalose/mannitol. Par exemple, la proportion de mannitol est d'environ 10%, environ 20%, environ 30%, environ 40%, environ 50%, environ 60%, environ 70%, environ 80% ou environ 90% en masse par rapport au mélange tréhalose/mannitol, de préférence environ 29% en masse par rapport au mélange tréhalose/mannitol.

Selon un mode particulier de réalisation, le procédé selon l'invention peut comprendre les étapes suivantes :
- la préparation des excipients adaptés à la dessiccation, lesdits excipients étant de préférence préparés en milieux aqueux ;
- le mélange des excipients adaptés à la dessiccation avec le complexe défini ci-dessus ; et
- la dessiccation, notamment par lyophilisation, du mélange ainsi obtenu.

Selon un mode de réalisation particulier, l'étape de congélation mise en œuvre au cours de la lyophilisation correspond à la congélation du mélange comprenant les excipients adaptés à la dessiccation et le complexe défini ci-dessus. La congélation est réalisée de manière à préserver l'activité biologique des vecteurs d'acides nucléiques, de préférence de manière à préserver l'assemblage supramoléculaire des vecteurs d'acides nucléiques. Les paramètres de congélation sont maîtrisés pour favoriser l'amorphisation et/ou limiter la croissance cristalline des constituants du mélange.

De préférence, la cinétique de congélation est rapide, celle-ci dépendant notamment de la température de congélation. De manière préférée, la vitesse de la congélation est comprise entre -40°C/min et -0,5°C/min.

De préférence, afin de favoriser l'amorphisation, la congélation est réalisée dans l'azote liquide, dont la température est notamment d'environ -190°C. De manière préférée, la durée de la congélation dans l'azote liquide est comprise entre 1 minute et 24 heures, en particulier entre 1 minute et 1 heure, ladite durée de congélation étant notamment d'environ 10 minutes.

Selon un mode de réalisation particulier, les paramètres du procédé de lyophilisation permettent de préserver l'activité biologique desdits complexes. En particulier, les paramètres opératoires qui pilotent le cycle de lyophilisation (température ambiante ou température étagère, pression dans la chambre de séchage et durée du cycle) sont choisis de façon appropriée pour conserver l'activité biologique des complexes, et peuvent être adaptés en fonction du volume du mélange aqueux comprenant lesdits complexes (plus particulièrement en fonction du volume d'eau à sublimer).

Selon un mode de réalisation particulier, la durée de lyophilisation comprenant la dessiccation primaire et la dessiccation secondaire est comprise entre 10 et 80 heures, préférentiellement entre 15 et 72 heures, plus préférentiellement entre 20 et 48 heures, de manière préférée environ 30 heures. La durée de lyophilisation peut être adaptée par l'homme du métier en fonction du volume et de la formulation du mélange aqueux à lyophiliser.

Selon un mode de réalisation particulier, la dessiccation, en particulier par lyophilisation, est réalisée à pression atmosphérique.

### • Etape b) de mélange :

Le procédé selon l'invention peut comprendre une étape destinée à casser la structure du produit obtenu après dessiccation. Cette étape peut être une étape de broyage léger (peu énergétique) ou de redispersion du produit. Elle peut être réalisée par exemple en utilisant une grille de vibration ou de calibrage, ou dans un mélangeur à pale par exemple lors du mélange avec le diluant.

Avant compression, le mélange sec, éventuellement broyé, est mélangé avec un ou plusieurs excipient(s) de compression. Par « excipient de compression » est entendu tout excipient :
- permettant de protéger le complexe entre l'acide nucléique et la particule lipidique durant l'étape c) de compression ; et/ou
- permettant de réduire les phénomènes de friction durant l'étape c) de compression ; et/ou
- permettant une alimentation régulière de la matrice utilisée pour la compression, la matrice délimitant le compartiment où s'effectue la compression de la poudre
- et/ou
- permettant d'obtenir un comprimé d'une masse suffisante et d'une résistance mécanique adéquate,
- et/ou
- permettant de contrôler ou de différer la libération du vecteur d'acide nucléique,
- et/ou
- permettant la désagrégation du comprimé.

Selon un mode de réalisation particulier, le/les excipient(s) de compression est/sont choisi(s) parmi les excipients utilisés dans des mélanges granulaires pour compression.

Dans un mode de réalisation préféré, l'excipient de compression est un lubrifiant, éventuellement mélangé avec au moins un autre excipient de compression bien connu de l'homme du métier, de préférence un diluant.

On entend par « lubrifiant », tout excipient permettant de réduire l'adhérence de la poudre au poinçon et à la matrice de la machine de compression, et/ou engendrant la diminution des frictions entre les particules et la matrice pendant la compression et l'éjection du comprimé. Selon un mode de réalisation particulier, le lubrifiant représente de 0,25 à 5% en masse de comprimé fini. De manière préférée, le lubrifiant représente environ 0,5% en masse de comprimé fini. Selon un mode de réalisation préféré, le lubrifiant est choisi parmi le stéarate de magnésium, le talc, le fumarate de stéaryle sodique et l'acide stéarique, éventuellement en mélange. De préférence, le lubrifiant est le stéarate de magnésium.

On entend par « diluant » tout excipient ayant un rôle de remplissage lorsque la quantité de principe actif et des autres excipients est insuffisante pour faire un comprimé de taille convenable, permettant ainsi un réglage de la masse et des dimensions du comprimé.

Selon un mode de réalisation préféré, le diluant est choisi parmi le lactose, l'amidon et ses dérivés, le phosphate de calcium et ses dérivés notamment le phosphate dicalcique et la cellulose et ses dérivés notamment la cellulose microcristalline, éventuellement en mélange. De préférence, le diluant est le lactose.

Le comprimé peut en outre contenir des excipients pharmaceutiques bien connus de l'homme de l'art tels que des agents d'écoulement, des délitants, des liants, des excipients permettant de contrôler ou de différer la libération des vecteurs d'acides nucléiques, des excipients conférant au comprimé des propriétés de muco-adhésion ainsi que leur combinaison. Par exemple, le comprimé peut contenir un ou plusieurs des excipients suivants : saccharose, glucose, mannitol, tréhalose et silice.

De manière préférée, le ou les excipient(s) de compression sont des excipients secs, de préférence sous forme de poudre.

Le procédé de l'invention peut comprendre une étape de mélange des différents excipients de compression préalablement à l'étape de mélange avec le mélange sec obtenu à l'issue de l'étape a) de dessiccation.

### • Etape c) de compression :

Le procédé selon l'invention comprend en outre une étape c) de compression du mélange obtenu à l'issue de l'étape b). L'étape de compression est réalisée dans des conditions permettant de préserver la fonction biologique des vecteurs d'acides nucléiques. En particulier, l'étape de compression est réalisée de manière à préserver l'activité thérapeutique des vecteurs d'acides nucléiques, par exemple l'inhibition de l'expression d'un gène cible, dans le cas des siRNA.

Les stress mécaniques appliqués durant l'étape de compression peuvent être contrôlés et suivis. Plus particulièrement, la pression appliquée au mélange comprenant les vecteurs d'acides nucléiques selon l'invention est contrôlée et suivie durant l'étape de compression. De manière préférée, la compression de l'étape c) est réalisée à une pression comprise entre 10 et 400 MPa, de préférence entre 50 et 250 MPa.

Selon un mode de réalisation préféré, la compression de l'étape c) est réalisée au moyen d'une presse à comprimer, par exemple une presse rotative ou une presse alternative.

Dans un mode de réalisation particulier, une étape de granulation humide ou de granulation sèche est réalisée en amont de l'étape c) de compression. Les étapes de « granulation humide » ou « granulation sèche » consistent en l'accroissement de la taille des particules dans le but d'obtenir des agglomérats, afin de faciliter la compression. Plus particulièrement, l'étape de granulation humide comprend l'ajout d'un agent liant permettant d'obtenir des granulés sous forme d'agglomérats qui sont ensuite séchés et éventuellement tamisés avant l'étape de compression. La granulation sèche consiste à comprimer fortement les particules pour obtenir un agglomérat avant de les broyer pour former des grains qui sont éventuellement calibrés avant l'étape de mise en forme finale par compression.

Selon un mode de réalisation préféré, l'étape c) de compression est une compression directe, la compression directe ne nécessitant pas d'étape préalable de granulation. Selon ce mode de réalisation, le mélange sec obtenu à l'issue de l'étape de dessiccation (étape a)), éventuellement mélangé avec des excipients de compression (étape b)) possède des propriétés adaptées pour la compression directe.

Dans un mode de réalisation particulier, le procédé selon l'invention comprend, après l'étape de compression, une étape de pelliculage (ou enrobage) du comprimé obtenu. L'intérêt de cette étape est la production d'un comprimé qui est mieux protégé, dont la stabilité est améliorée, et/ou dont la cinétique de libération est adaptée. La nature des agents de pelliculage et/ou d'enrobage et le procédé utilisé pour mettre en œuvre cette étape sont bien connus de l'homme du métier formulateur.

Selon un deuxième aspect, l'invention concerne un comprimé susceptible d'être obtenu au moyen du procédé tel que décrit ci-dessus.

En particulier, l'invention concerne un comprimé comprenant :
- un ou plusieurs vecteur(s) d'acide nucléique (ou complexe(s)) tel que décrit ci-dessus ;
- un ou plusieurs excipient(s) adapté(s) à la dessiccation ;
- un ou plusieurs excipient(s) de compression.

En particulier, le comprimé de l'invention peut comprendre :
- un complexe entre un acide nucléique et un liposome ou une micelle comprenant un lipide cationique;
- un ou plusieurs excipients de lyophilisation appropriés permettant de protéger ledit complexe durant l'étape a) de lyophilisation et/ou permettant d'obtenir un lyophilisat de texture adaptée pour l'étape de compression, de préférence du tréhalose et du mannitol ;
- un ou plusieurs excipients de compression appropriés permettant de protéger ledit complexe durant l'étape c) de compression et de conserver l'activité biologique du complexe.

En particulier, le comprimé fini comprend de 1 µg à 1 mg d'acide nucléique, de préférence environ 10 µg.

La masse de lipide cationique dans le comprimé fini peut être comprise entre 18,7 µg et 18,7 mg, de préférence environ 170 µg.

La masse de lipide neutre dans le comprimé fini peut être comprise entre 16,5 µg et 16,5 mg, de préférence environ 150 µg.

La masse de polymère dans le comprimé fini peut être comprise entre 1 µg et 1 mg, de préférence environ 10 µg.

La masse des excipients adaptés à la dessiccation peut être comprise dans le comprimé fini entre 36 mg et 720 mg, de préférence environ 126 mg. En particulier, la masse de tréhalose dans le comprimé fini peut être comprise entre 3,6 mg et 720 mg, de préférence environ 90 mg. En particulier, la masse de mannitol dans le comprimé fini peut être comprise entre 0 mg et 648 mg, de préférence environ 36 mg.

La proportion des excipients adaptés à la compression peut être comprise entre 0,25% et 95%, en pourcentage massique par rapport au comprimé fini. En particulier, la proportion de lubrifiant, étant de préférence du stéarate de magnésium, peut être comprise entre 0,25% et 5%, en pourcentage massique par rapport au comprimé fini, de préférence environ 0,5%. En particulier, la proportion des autres excipients de compression, peut être comprise entre 0% et 94,75%, en pourcentage massique par rapport au comprimé fini.

Selon un mode particulier de réalisation, le comprimé selon l'invention comprend :
- un complexe entre un acide nucléique et un liposome ou une micelle comprenant du DMAPAP et du DOPE, le complexe comprenant en outre de l'alginate, notamment de l'alginate de sodium ;
- du tréhalose et du mannitol ; et
- du lactose.

Selon une variante de ce mode de réalisation, le comprimé ainsi composé présente, après remise en suspension dans l'eau, un profil SAXS (Small Angle X Ray Scattering) dans un rayonnement synchrotron comprenant une phase cubique et une phase lamellaire, et éventuellement une phase cristalline liquide. Le profil SAXS peut être déterminé selon le protocole présenté dans les exemples. Le profil SAXS du comprimé selon l'invention après remise en suspension dans l'eau présente notamment des pics à q=0,086 nm⁻¹ et q=0,101 nm⁻¹. Selon un mode particulier, la phase lamellaire est majoritaire par rapport à la phase cubique.

Selon un mode de réalisation particulier, le comprimé selon l'invention est destiné à une administration par voie orale, vaginale, rectale, sublinguale ou transmucosale. De préférence, le comprimé selon l'invention est destiné à une administration par voie orale.

Selon un autre aspect, la présente invention concerne le comprimé de l'invention pour une utilisation dans le traitement des pathologies locales, loco-régionales ou dans le traitement des pathologies systémiques. De préférence, le comprimé selon l'invention est utilisé dans le traitement des pathologies du tractus gastro-intestinal. Par exemple, les pathologies du tractus gastro-intestinal peuvent être la maladie de Crohn (l'acide nucléique étant par exemple un siRNA inhibant des protéines inflammatoires telles que TNFalpha), la rectocolite hémorragique (l'acide nucléique étant par exemple un oligonucléotide anti-ICAM-1, un plasmide codant l'IL10 ou un siRNA anti-TNFalpha), ou un cancer gastrique (l'acide nucléique étant par exemple un siRNA ou un oligonucléotide anti Her2).Le comprimé selon l'invention peut également être utilisé dans le traitement de pathologies vaginales, notamment la candidose vulvovaginale ou une infection au HSV, HPV ou HIV (via l'administration de siRNA antiviraux par exemple).

En outre, le comprimé peut être utilisé dans le traitement de pathologies telles que l'amyotrophie spinale (l'acide nucléique étant par exemple l'oligonucléotide antisens Nusinersen), l'hypercholestérolémie familiale (l'acide nucléique étant par exemple l'oligonucléotide antisens Mipomersen), la myopathie de Duchenne, ou encore la dégénérescence maculaire liée à l'âge (l'acide nucléique étant par exemple l'aptamère Macugen).

Le comprimé selon l'invention est stable, c'est-à-dire qu'il conserve substantiellement son activité biologique, de préférence sur une durée pouvant atteindre 2 semaines, 1 mois, 3 mois, 1 an, à température ambiante (entre la formulation du comprimé et l'administration).

### EXEMPLES

### Exemple 1 : Préparation du comprimé

### 1. Formation des lipoplexes

La première étape consiste en la formation de lipoplexe, c'est à dire du complexe entre l'acide nucléique et le liposome. L'acide nucléique utilisé dans cet exemple est un siRNA spécifique de la luciférase. Le liposome utilisé ici comprend un lipide cationique (le DMAPAP) et un lipide neutre (le DOPE). La formation des lipoplexes a été réalisée au moyen des constituants suivants :

| | |
|---|---|
| siRNA luciférase ou siRNA contrôle | 1,33 µg/µL |
| liposome (DMAPAP, DOPE) | 20mM |
| polymère (alginate de sodium) | 1,5 µg/µL |
| NaCl | 150mM dans eau milliQ |

Les préparations de siRNA, de liposome et de polymère ont été vortexées.
- Le liposome a été mélangé avec la solution de NaCl pour obtenir un volume final de 200µL (10,7µL de liposome avec 189µL de solution de NaCl).
- D'autre part, le siRNA a été mélangé avec le polymère dans une solution de NaCl pour obtenir un volume final de 200µL (7,5µL de siRNA + 6,5µL de polymère + 186µL de solution de NaCl). Le mélange obtenu comprend donc un ratio entre le siRNA et le polymère de 1:1 en masse.
- Les deux préparations obtenues, de 200µl chacune, ont été mélangées, vortexées, puis laissées 30min à température ambiante pour permettre la formation du complexe entre le siRNA et le liposome.

Les constituants ci-dessus ont été mélangés de manière à former un lipoplexe dont le rapport de charge +/- est de 8.

### 2. Lyophilisation

Dans cet exemple, le mannitol et le tréhalose ont été utilisés comme excipients adaptés à la lyophilisation.

La suspension de lipoplexe précédemment obtenue a été transférée dans un tube à centrifuger de 50mL. Une solution comprenant 2,5% m/v de tréhalose et 1% m/v de mannitol a été préparée. 3,6 mL de cette solution tréhalose/mannitol ont ensuite été ajoutés aux 400µL de la suspension de lipoplexe. Le tube a ensuite été plongé dans l'azote liquide durant 10 minutes. La lyophilisation a été réalisée sur un lyophilisateur comprenant un hérisson équipé de 12 robinets sur lesquels sont branchés les tubes d'échantillons congelés. La lyophilisation a été réalisée durant 30 heures, à une pression dans la chambre de lyophilisation inférieure à 0,2 mbar (plus particulièrement entre 0,1 et 0,18 mbar). L'apport d'énergie nécessaire à la compensation de l'énergie absorbée durant la sublimation est fourni par l'air ambiant (température ambiante, plus particulièrement entre 20 et 25°C).

Des sondes de température ont été placées dans les échantillons pour suivre la température du produit pendant le déroulement du procédé de congélation (cf figure 1b) et de lyophilisation (cf figure la).

### 3. Compression

Dans cet exemple, le stéarate de magnésium (lubrifiant) et le lactose (diluant) ont été utilisés comme excipients adaptés à la compression.

Le lyophilisat obtenu précédemment a d'abord été réduit en poudre par broyage léger dans un mortier. Puis 36,2 mg de lactose et 0,8 mg de stéarate de magnésium ont été ajoutés dans le mortier et mélangés au lyophilisât.

La compression a été réalisée au moyen d'un simulateur de compression StylOne Evolution selon les paramètres suivants :
- Poinçons plats chanfreinés de 6mm de diamètre
- Cycle de compression = cycle 1 compression (cycle par défaut), vitesse 2%
- Pilotage en force
- Dosage (hauteur de remplissage de la matrice)=18mm
- Remplissage manuel
- Force de compression = 1,4 à 7,1 kN (soit 50 à 250 MPa)

### Exemple 2 : Exemples de composition de comprimés contenant des lipoplexes

Le tableau 1 suivant est présenté afin d'illustrer la composition des comprimés contenant des lipoplexes. Les différents constituants sont exprimés en mg.

**Tableau 1. Exemples de composition de comprimés contenant des lipoplexes.**

| | Comprimé 1 | Comprimé 2 | Comprimé 3 |
|---|---|---|---|
| Acide nucléique | 0,01 mg | 0,10 mg | 0,01 mg |
| DMAPAP | 0,17 mg | 1,70 mg | 0,17 mg |
| DOPE | 0,15 mg | 1,50 mg | 0,15 mg |
| Alginate | 0,01 mg | 0,10 mg | 0,01 mg |
| Tréhalose | 90,00 mg | 90,00 mg | 90,00 mg |
| Mannitol | 36,00 mg | 36,00 mg | 36,00 mg |
| Stéarate de Mg | 0,75 mg | 0,75 mg | 2,25 mg |
| Lactose | 22,91 mg | 19,85 mg | 126,34 mg |

### Exemple 3 : Test de l'efficacité du comprimé

L'efficacité des comprimés est ensuite évaluée sur des cellules en culture exprimant le gène de la luciférase. Lorsque ces cellules sont mises en présence de vecteurs contenant des siRNA spécifiques de la luciférase, l'activité luciférase est inhibée. Le test consiste à transfecter des cellules de mélanome de souris exprimant la luciférase (B16-Luc) et à doser 48h plus tard l'activité luciférase résiduelle dans les cellules. Rapportée à l'activité de cellules non traitées ou traitées avec des vecteurs contenant des siRNA contrôle (sans effet inhibiteur), cette mesure permet d'obtenir le pourcentage d'inhibition induit par le vecteur de siRNA étudié.

Pour évaluer le maintien de cette activité après compression, les comprimés sont solubilisés avant d'être mis en contact avec les cellules à transfecter. Les lyophilisats (obtenus avant l'étape de compression) ont également été réhydratés pour analyser l'efficacité du mélange sec avant compression.

Le test a donc consisté à traiter les cellules B16luc avec :
- Un témoin négatif : c'est-à-dire des lipoplexes contenant un siRNA contrôle (dont la séquence a été choisie de façon à ce qu'il n'inhibe aucune cible protéique), remis en suspension depuis une forme comprimée;
- Un témoin positif de transfection : c'est-à-dire les lipoplexes en suspension (non lyophilisés, non comprimés) ;
- Un lyophilisat réhydraté ; ou
- Un comprimé réhydraté.

Plus précisément, les lipoplexes en suspension (correspondant au témoin positif de transfection) ont été préparés tel que décrit ci-dessus, puis mélangés avec du milieu de culture. Le milieu de culture correspond à du milieu complet (DMEM + Glutamax + sérum de veau fœtal + pénicilline/streptomycine) supplémenté en Généticine (G418).

Le lyophilisat et le comprimé ont été préparés tel que décrit ci-dessus, puis dissous dans de l'eau distillée ou milliQ et mélangés avec du milieu de culture.

La manipulation s'effectue sur 4 jours (J0, J1, J2 et J3) :
A J0, les cellules sont préparées sur plaque 24 puits (40000 cellules/puits).
A J1, le milieu de culture des cellules est remplacé par 1mL de milieu de transfection (de façon à ajouter 0,5 µg de siRNA par puits). Le milieu de transfection correspond au milieu comprenant le témoin négatif, le témoin positif de transfection, le lyophilisat ou le comprimé.
A J2, le milieu de transfection est remplacé par du milieu de culture (milieu complet + G418).
A J3, les cellules sont lysées et l'activité luciférase dosée.
Le test est effectué en triplicat (3 répétitions par condition).

### Résultats :

### Test de l'efficacité du comprimé

Les tests de transfection sur cellules ont mis en évidence une bonne efficacité des comprimés contenant les lipoplexes (efficacité exprimée par le pourcentage d'inhibition des comprimés par rapport à des cellules non transfectées). De plus, sur toute la gamme de contraintes de compression appliquées, il n'a pas été observé d'effet de la contrainte de compression sur le pourcentage d'inhibition du gène de la luciférase (Figure 2).

### Exemple 4 : structure supramoléculaire

La technique SAXS (Small Angle X Ray Scattering) a été utilisée pour identifier la structure supramoléculaire des vecteurs de siRNA, à la fois sur des particules en suspension ou sur des formes sèches, comme un lyophilisat ou un comprimé. Les siRNA utilisés sont des siRNA de contrôle, et chaque échantillon comprend 100 microgrammes de siRNA. Cette identification est rendue nécessaire par le fait que la structure de ce type de particules est directement reliée à leur efficacité d'administration d'acide nucléique. L'analyse SAXS dans un rayonnement synchrotron est la seule technologie donnant accès à cette identification de structure dans des formes galéniques variées, en suspension dans un milieu aqueux ou dans une poudre.

Dans un premier temps, la structure de lipoplexes de siRNA (DMAPAP/DOPE + alginate) préparés à un rapport de charge +/- de 8 a été déterminée. L'étude réalisée sur les lipoplexes en suspension dans un tampon salin (NaCl 150 mM) a permis de montrer que ces lipoplexes se présentaient sous la forme de deux structures coexistant, une phase lamellaire et une phase cubique (Figure 3).

Nous avons ensuite étudié le devenir de cette structure lorsque les vecteurs en suspension subissaient une étape de lyophilisation, étape préalable nécessaire avant de pouvoir effectuer l'étape de compression, puis après l'étape de compression. La Figure 4 montre que l'on détecte un signal pour les deux types d'échantillon (lyophilisât et comprimé), avec un pic unique plus ou moins large à q=0,096 nm⁻¹. Sa position, intermédiaire entre les valeurs des pics L₁ et C₁ du lipoplexe en suspension (Figure 3), et son unicité ne permettent pas de déduire la phase correspondant à ce pic. Le pic observé pour l'échantillon lyophilisé est plus intense que celui de l'échantillon comprimé, mais ce dernier reste néanmoins parfaitement détectable.

Les étapes de lyophilisation et de compression nécessitent d'ajouter des excipients. Nous avons donc évalué les signaux obtenus pour des lipoplexes dilués dans un mélange NaCl / excipients dans les mêmes conditions de concentration que les échantillons lyophilisés et comprimés (Figure 5). Les profils obtenus indiquent la présence d'une phase cubique unique, dont le premier pic est légèrement décalé entre les conditions « lyophilisât » et « comprimé ». La position de ce pic est voisine du pic détecté lors de l'analyse des poudres, ce qui pourrait indiquer que la phase présente dans les formes sèches est une phase cubique. Le pic correspondant à la phase lamellaire n'est plus détectable, ni dans les formes sèches, ni dans les suspensions, en présence d'excipients.

Nous avons ensuite analysé le profil SAXS des échantillons lyophilisés ou comprimés remis en suspension. L'analyse précédente ayant montré l'influence de la présence des excipients sur la structure des particules (Figure 5), nous avons comparé chaque échantillon resuspendu avec des lipoplexes en suspension dans les mêmes conditions de concentration en excipients. Les résultats obtenus sont présentés dans la Figure 6. On constate que le profil SAXS de l'échantillon lyophilisé resuspendu est proche du profil de lipoplexes en suspension dans les mêmes conditions (Figure 6A), ce qui suggère que la lyophilisation ne modifie pas la structure des lipoplexes. En présence d'excipient, on ne détecte qu'une phase cubique. En revanche, pour l'échantillon comprimé resuspendu, le profil obtenu permet de détecter nettement deux phases, une lamellaire et une cubique, dont les pics principaux ont des positions voisines de ceux obtenus pour la suspension initiale de lipoplexes (Figure 3). Le profil de cet échantillon est nettement différent de celui d'une suspension de lipoplexes placée dans des conditions identiques de concentration en excipient. Il est également différent de celui de la suspension initiale de lipoplexes, puisque la proportion relative des phases cubique et lamellaire est inversée. Dans la suspension initiale, la phase cubique est majoritaire, dans l'échantillon comprimé remis en suspension, c'est la phase lamellaire qui est majoritaire. Ce résultat suggère que la compression a une influence sur la structure en modifiant la proportion relative d'une phase par rapport à l'autre, sans toutefois faire apparaître une nouvelle phase ou en faire disparaitre.

### Exemple 5 : évaluation de l'efficacité in vivo

**Modèle** : induction de la colite ulcérative chez la souris par administration de sulfate de dextran (SD) dans l'eau de boisson pendant 7 jours.

**Evaluation de l'efficacité des vecteurs** : administration orale de comprimés contenant des lipoplexes de siRNA. Les comprimés sont donnés à J1, J3 et J5 à des souris sous traitement SD (commencé à J0). Chaque souris reçoit un comprimé contenant 50µg de siRNA formulé en lipoplexes.

Trois groupes de 10 souris sont utilisés :
- Groupe 1 : boisson eau, pas d'administration de lipoplexes
- Groupe 2 : boisson SD, à J1, 3 et 5, comprimé contenant des lipoplexes de siRNA dirigé contre TNF alpha
- Groupe 3 : boisson SD, à J1, 3 et 5, comprimé contenant des lipoplexes de siRNA contrôle (sans effet inhibiteur)
- Groupe 4 : boisson SD, pas d'administration de lipoplexes

Pendant le traitement, suivi du poids et aspect des fèces (présence de sang). A J7, euthanasie, prélèvement du colon, mesure de la taille du colon, dosage de cytokines inflammatoires sur un lysat de colon, analyse histologique de l'inflammation et de l'atteinte au tissu sur des coupes de colon.

### Résultat attendu :

Diminution voire disparition des signes cliniques et biochimiques de la maladie suite au traitement. Cette diminution est spécifique de la séquence du siRNA, c'est-à-dire être observée chez les souris traitées par les lipoplexes contenant un siRNA anti TNF alpha et pas chez celles traitées avec des lipoplexes contenant un siRNA contrôle.

## Revendications

1. Procédé de fabrication d'un comprimé comprenant un complexe entre :
(i) un acide nucléique; et
(ii) une particule lipidique comprenant un lipide cationique, la particule lipidique étant de préférence un liposome ou une micelle, de préférence un liposome ;
ledit procédé comprenant les étapes suivantes:
a) la dessiccation, notamment par lyophilisation, d'un mélange aqueux comprenant:
- un complexe entre (i) un acide nucléique et (ii) une particule lipidique comprenant un lipide cationique, la particule lipidique étant de préférence un liposome ou une micelle, de préférence un liposome ;
- un ou plusieurs excipient(s) adapté(s) à la dessiccation, notamment adaptés à la lyophilisation ;
b) le mélange d'un ou plusieurs excipient(s) de compression avec le mélange sec obtenu à l'étape a); et
c) la compression du mélange obtenu à l'étape b).

2. Procédé selon la revendication 1, **caractérisé en ce que** le lipide cationique est choisi parmi les lipopolyamines, les ammoniums quaternaires, et les lipides ayant une tête cationique de type guanidine ou imidazole, de préférence le lipide cationique est choisi parmi le Di-Myristylaminopropylaminopropyl (DMAPAP), le N-[1-(2,3-dioleyloxy)propyl]-N,N,N-trimethylammonium chloride (DOTMA), le [1,2-bis(oleoyloxy)-3-(trimethylammonio)propane] (DOTAP), le 3β[N-(N',N'-dimethylaminoethane)-carbamoyl] cholesterol (DCChol) et le dioctadecylamidoglycylspermine (DOGS), de préférence le Di-Myristylaminopropylaminopropyl (DMAPAP) ;
le lipide cationique étant éventuellement en mélange avec un lipide neutre, notamment choisi parmi le 1,2-dioleoyl-sn-glycero-3-phosphoethanolamine (DOPE), le Cholestérol, le dioleoyl-sn-glycero-3-phosphocholine (DOPC), le dipalmitoyl-sn-glycero-3-phosphocholine (DPPC) et le N'-(rac-1-[1-(F-octyl)undec-10-enyl]-2-(hexadecyl)glycero-3-phosphoethanoyl)-sperminecarboxamide), de préférence le lipide neutre est le 1,2-dioleoyl-sn-glycero-3-phosphoethanolamine (DOPE).

3. Procédé selon la revendication 2, dans lequel le mélange entre le lipide cationique et le lipide neutre comprend :
- entre 50% et 99% en mole de lipide cationique dans le mélange, de préférence environ 50% ;
- entre 1% et 50% en mole de lipide neutre dans le mélange, de préférence environ 50%.

4. Procédé selon l'une quelconque des revendications précédentes, l'acide nucléique étant un acide nucléique thérapeutique, notamment un siRNA, un miRNA, un shRNA, un plasmide ou un ARNm.

5. Procédé selon l'une quelconque des revendications précédentes, **caractérisé en ce que** le rapport de charge (+/-) entre les charges positives du lipide cationique et les charges négatives de l'acide nucléique est compris entre 0,5 et 10, de préférence entre 4 et 10, de préférence environ 8.

6. Procédé selon l'une quelconque des revendications précédentes, **caractérisé en ce que** le ou les excipient(s) adapté(s) à la lyophilisation sont des agents protecteurs (cryoprotecteurs ou lyoprotecteurs) et/ou des agents de charge, notamment choisis parmi le tréhalose, le mannitol, le saccharose, le sorbitol, le lactose, le glucose, le glycérol, la glycine, l'alanine, la lysine, le polyéthylène glycol, la poly vinyl pyrrolidone et le dextran, éventuellement en mélange.

7. Procédé selon la revendication 6, **caractérisé en ce que** l'excipient adapté à la lyophilisation est le tréhalose, notamment en une proportion comprise entre 10% et 100% en masse par rapport à la masse d'excipient(s) adapté(s) à la lyophilisation, éventuellement mélangé avec un autre excipient adapté à la lyophilisation, de préférence le mannitol, notamment en une proportion comprise entre 0 et 90% en masse par rapport à la masse d'excipient(s) adapté(s) à la lyophilisation.

8. Procédé selon l'une quelconque des revendications précédentes, **caractérisé en ce que** le ou les excipient(s) de compression permettent de protéger le complexe entre l'acide nucléique et la particule lipidique comprenant le lipide cationique durant l'étape c) de compression, ledit excipient de compression étant en particulier un lubrifiant (par exemple le stéarate de magnésium, l'acide stéarique et le talc, de préférence le stéarate de magnésium), éventuellement mélangé avec un autre excipient de compression, de préférence un diluant (par exemple le lactose, l'amidon, le phosphate de calcium et ses dérivés et la cellulose et ses dérivés, de préférence le lactose).

9. Procédé selon la revendication 8, **caractérisé en ce que** le lubrifiant représente de 0,25 à 5% en masse du comprimé fini, de préférence 0,5%.

10. Procédé selon l'une quelconque des revendications précédentes, **caractérisé en ce que** la compression de l'étape c) est réalisée à une pression comprise entre 10 et 400 MPa, de préférence entre 50 et 250 MPa.

11. Comprimé susceptible d'être obtenu au moyen du procédé selon l'une quelconque des revendications 1 à 10.

12. Comprimé comprenant:
- un complexe entre un acide nucléique et un liposome ou une micelle comprenant un lipide cationique;
- un ou plusieurs excipients de lyophilisation appropriés permettant de protéger ledit complexe durant l'étape a) de lyophilisation et/ou permettant d'obtenir un lyophilisat de texture adaptée pour l'étape de compression, de préférence du tréhalose et du mannitol.
- un ou plusieurs excipients de compression appropriés permettant de protéger ledit complexe durant l'étape c) de compression et de conserver l'activité biologique du complexe.

13. Comprimé selon la revendication 11 ou 12, étant destiné à une administration par voie orale, vaginale, rectale, sublinguale ou transmucosale, de préférence par voie orale.

14. Comprimé selon l'une quelconque des revendications 11 à 13 pour une utilisation dans le traitement des pathologies du tractus gastro-intestinal ou des pathologies vaginales.

## Patentansprüche

1. Verfahren zur Herstellung einer Tablette, umfassend einen Komplex zwischen:
(i) einer Nukleinsäure; und
(ii) einem Lipidpartikel, umfassend ein kationisches Lipid, wobei das Lipidpartikel vorzugsweise ein Liposom oder eine Mizelle, vorzugsweise ein Liposom ist;
wobei das Verfahren die folgenden Schritte umfasst:
a) Trocknung, insbesondere mittels Lyophilisierung, eines wässrigen Gemisches, umfassend:
- einen Komplex zwischen (i) einer Nukleinsäure und (ii) einem Lipidpartikel, umfassend ein kationisches Lipid, wobei das Lipidpartikel vorzugsweise ein Liposom oder eine Mizelle, vorzugsweise ein Liposom ist;
- einen oder mehrere Exzipienten, die für die Trocknung geeignet sind, insbesondere für die Lyophilisierung geeignet sind;
b) Mischen eines oder mehrerer verpressbarer Exzipienten mit dem in Schritt a) erhaltenen trockenen Gemisch; und
c) Verpressen des in Schritt b) erhaltenen Gemisches.

2. Verfahren gemäß Anspruch 1, **dadurch gekennzeichnet, dass** das kationische Lipid aus Lipopolyaminen, quartären Ammoniumverbindungen und Lipiden, die einen kationischen Guanidin- oder Imidazol-Kopf besitzen, ausgewählt ist, vorzugsweise das kationische Lipid aus Dimyristylaminopropylaminopropyl (DMAPAP), N-[1-(2,3-Dioleyloxy)propyl]-N,N,N-trimethylammoniumchlorid (DOTMA), [1,2-Bis(oleoyloxy)-3-(trimethylammonio)propan] (DOTAP), 3β[N-(N',N'-Dimethylaminoethan)-carbamoyl]cholesterol (DCChol) und Dioctadecylamidoglycylspermin (DOGS), vorzugsweise aus Dimyristylaminopropylaminopropyl (DMAPAP) ausgewählt ist;
wobei das kationische Lipid gegebenenfalls in einem Gemisch mit einem neutralen Lipid vorliegt, das insbesondere aus 1,2-Dioleoyl-sn-glycero-3-phosphoethanolamin (DOPE), Cholesterol, Dioleoyl-sn-glycero-3-phosphocholin (DOPC), Dipalmitoyl-sn-glycero-3-phosphocholin (DPPC) und N-(raz.-1-[1]-(F-Octyl)undec-10-enyl]-2-(hexadecyl)glycero-3-phosphoethanoyl)spermincarboxamid) ausgewählt ist, vorzugsweise das neutrale Lipid 1,2-Dioleoyl-sn-glycero-3-phosphoethanolamin (DOPE) ist.

3. Verfahren gemäß Anspruch 2, wobei das Gemisch aus dem kationischen Lipid und dem neutralen Lipid umfasst:
- zwischen 50 Mol-% und 99 Mol-% kationisches Lipid in dem Gemisch, vorzugsweise ungefähr 50 Mol-%;
- zwischen 1 Mol-% und 50 Mol-% neutrales Lipid in dem Gemisch, vorzugsweise ungefähr 50 Mol-%.

4. Verfahren gemäß einem der vorhergehenden Ansprüche, wobei die Nukleinsäure eine therapeutische Nukleinsäure, insbesondere eine siRNA, eine miRNA, eine shRNA, ein Plasmid oder eine mRNA ist.

5. Verfahren gemäß einem der vorhergehenden Ansprüche, **dadurch gekennzeichnet, dass** das Ladungsverhältnis (+/-) von den positiven Ladungen des kationischen Lipids zu den negativen Ladungen der Nukleinsäure zwischen 0,5 und 10, vorzugsweise zwischen 4 und 10, vorzugsweise ungefähr 8 beträgt.

6. Verfahren gemäß einem der vorhergehenden Ansprüche, **dadurch gekennzeichnet, dass** der oder die für die Lyophilisierung geeigneten Exzipienten Schutzmittel (Kryoschutzmittel oder Lyoschutzmittel) und/oder Füllstoffe sind, die insbesondere aus Trehalose, Mannit, Saccharose, Sorbit, Lactose, Glucose, Glycerol, Glycin, Alanin, Lysin, Polyethylenglycol, Polyvinylpyrrolidon und Dextran, gegebenenfalls als Gemisch ausgewählt sind.

7. Verfahren gemäß Anspruch 6, **dadurch gekennzeichnet, dass** der für die Lyophilisierung geeignete Exzipient Trehalose ist, insbesondere in einem Verhältnis zwischen 10 Gew.-% und 100 Gew.-% bezogen auf das Gewicht des oder der für die Lyophilisierung geeigneten Exzipienten, gegebenenfalls gemischt mit einem weiteren für die Lyophilisierung geeigneten Exzipienten, vorzugsweise Mannit, insbesondere in einem Verhältnis zwischen 0 und 90 Gew.-% bezogen auf das Gewicht des oder der für die Lyophilisierung geeigneten Exzipienten.

8. Verfahren gemäß einem der vorhergehenden Ansprüche, **dadurch gekennzeichnet, dass** der oder die verpressbaren Exzipienten erlauben, den Komplex zwischen der Nukleinsäure und dem Lipidpartikel, umfassend das kationische Lipid, während des Verpressungsschrittes c) zu schützen, wobei der verpressbare Exzipient insbesondere ein Gleitmittel (zum Beispiel Magnesiumstearat, Stearinsäure und Talkum, vorzugsweise Magnesiumstearat) ist, gegebenenfalls gemischt mit einem weiteren verpressbaren Exzipienten, vorzugsweise einem Verdünnungsmittel (zum Beispiel Lactose, Stärke, Calciumphosphat und Derivaten davon und Cellulose und Derivaten davon, vorzugsweise Lactose).

9. Verfahren gemäß Anspruch 8, **dadurch gekennzeichnet, dass** das Gleitmittel 0,25 Gew.-% bis 5 Gew.-%, vorzugsweise 0,5 Gew.-% der fertigen Tablette ausmacht.

10. Verfahren gemäß einem der vorhergehenden Ansprüche, **dadurch gekennzeichnet, dass** der Verpressungsschritt c) mit einem Druck zwischen 10 und 400 MPa, vorzugsweise zwischen 50 und 250 MPa durchgeführt wird.

11. Tablette, die mithilfe des Verfahrens gemäß einem der Ansprüche 1 bis 10 erhalten werden kann.

12. Tablette, umfassend
- einen Komplex zwischen einer Nukleinsäure und einem Liposom oder einer Mizelle, umfassend ein kationisches Lipid;
- einen oder mehrere Exzipienten, vorzugsweise Trehalose und Mannit, die für eine Lyophilisierung geeignet sind und erlauben, den Komplex während des Lyophilisierungsschrittes a) zu schützen, und/oder erlauben, ein Lyophilisat mit einer Textur zu erhalten, die für den Verpressungsschritt geeignet ist;
- einen oder mehrere Exzipienten, die zum Verpressen geeignet sind und erlauben, den Komplex während des Verpressungsschrittes c) zu schützen und die biologische Aktivität des Komplexes zu konservieren.

13. Tablette gemäß Anspruch 11 oder 12, die für eine orale, vaginale, rektale, sublinguale oder transmukosale, vorzugsweise eine orale Verabreichung bestimmt ist.

14. Tablette gemäß einem der Ansprüche 11 bis 13 zur Verwendung in der Behandlung von Erkrankungen des Magen-Darm-Trakts oder von vaginalen Erkrankungen.

## Claims

1. Process for the manufacture of a tablet comprising a complex between:
(i) a nucleic acid; and
(ii) a lipid particle comprising a cationic lipid, the lipid particle being preferably a liposome or a micelle, preferably a liposome;
said process comprising the following steps:
a) drying, in particular by freeze-drying, an aqueous mixture comprising:
- a complex between (i) a nucleic acid and (ii) a lipid particle comprising a cationic lipid, the lipid particle being preferably a liposome or a micelle, preferably a liposome;
- one or more excipient(s) suitable for drying, in particular for freeze-drying;
b) mixing one or more compression excipient(s) with the dry mixture obtained in step a); and
c) compressing the mixture obtained in step b).

2. Process according to claim 1, **characterized in that** the cationic lipid is selected from lipopolyamines, quaternary ammoniums, and lipids having a cationic head of the guanidine or imidazole type, preferably the cationic lipid is selected from Di-Myristylaminopropylaminopropyl (DMAPAP), N-[1-(2,3-dioleyloxy)propyl]-N,N,N-trimethylammonium chloride (DOTMA), [1,2-bis(oleoyloxy)-3-(trimethylammonio)propane] (DOTAP), 3β[N-(N',N'-dimethylaminoethane)-carbamoyl]cholesterol (DCChol) and dioctadecylamidoglycylspermine (DOGS), preferably Di-Myristylaminopropylaminopropyl (DMAPAP);
the cationic lipid being optionally mixed with a neutral lipid, in particular selected from 1,2-dioleoyl-sn-glycero-3-phosphoethanolamine (DOPE), cholesterol, dioleoyl-sn-glycero-3-phosphocholine (DOPC), dipalmitoyl-sn-glycero-3-phosphocholine (DPPC) and N'-(rac-1-[1-(F-octyl)undec-10-enyl]-2-(hexadecyl)glycero-3-phosphoethanoyl)-sperminecarboxamide), preferably the neutral lipid is 1,2-dioleoyl-sn-glycero-3-phosphoethanolamine (DOPE).

3. Process according to claim 2, wherein the mixture between the cationic lipid and the neutral lipid comprises:
- between 50 and 99 mol% cationic lipid in the mixture, preferably about 50 mol%;
- between 1 and 50 mol% neutral lipid in the mixture, preferably about 50 mol%.

4. Process according to any one of the preceding claims, the nucleic acid being a therapeutic nucleic acid, in particular an siRNA, an miRNA, an shRNA, a plasmid or an mRNA.

5. Process according to any one of the preceding claims, **characterized in that** the charge ratio (+/-) between the positive charges of the cationic lipid and the negative charges of the nucleic acid is between 0.5 and 10, preferably between 4 and 10, preferably about 8.

6. Process according to any one of the preceding claims, **characterized in that** the excipient(s) suitable for freeze-drying are protective agents (cryoprotectants or lyoprotectants) and/or fillers, in particular selected from trehalose, mannitol, sucrose, sorbitol, lactose, glucose, glycerol, glycine, alanine, lysine, polyethylene glycol, polyvinyl pyrrolidone and dextran, optionally in mixture.

7. Process according to claim 6, **characterized in that** the excipient suitable for freeze-drying is trehalose, in particular in a proportion of between 10 and 100 mass% of the excipient(s) suitable for freeze-drying, optionally mixed with another excipient suitable for freeze-drying, preferably mannitol, in particular in a proportion of between 0 and 90 mass% of the excipient(s) suitable for freeze-drying.

8. Process according to any one of the preceding claims, **characterized in that** the compression excipient(s) protect the complex between the nucleic acid and the lipid particle comprising the cationic lipid during the compression step c), said compression excipient being in particular a lubricant (for example magnesium stearate, stearic acid and talc, preferably magnesium stearate), optionally mixed with another compression excipient, preferably a diluent (for example lactose, starch, calcium phosphate and derivatives thereof and cellulose and derivatives thereof, preferably lactose).

9. Process according to claim 8, **characterized in that** the lubricant represents from 0.25 to 5 mass% of the finished tablet, preferably 0.5%.

10. Process according to any one of the preceding claims, **characterized in that** the compression of step c) is carried out at a pressure between 10 and 400 MPa, preferably between 50 and 250 MPa.

11. Tablet obtainable by the process according to any one of claims 1 to 10.

12. Tablet comprising:
- a complex between a nucleic acid and a liposome or a micelle comprising a cationic lipid;
- one or more suitable freeze-drying excipients to protect said complex during the freeze-drying step a) and/or to obtain a freeze-dried product of a texture suitable for the compression step, preferably trehalose and mannitol.
- one or more suitable compression excipients to protect said complex during the compression step c) and to preserve the biological activity of the complex.

13. Tablet according to claim 11 or 12, for oral, vaginal, rectal, sublingual or transmucosal administration, preferably for oral administration.

14. Tablet according to any one of claims 11 to 13 for use in the treatment of gastrointestinal tract or vaginal disorders.
